# FASCICULE DE BREVET EUROPEEN

(11) **EP 0 584 006 B1**
(45) Date de publication et mention de la délivrance du brevet: **05.06.1996**
(21) Numéro de dépôt: 93402028.0
(22) Date de dépôt: 06.08.1993
(51) Int. Cl.: C07C 2/58

(54) **Procédé d'alkylation de paraffines**
Verfahren zur Alkylierung von Paraffinen
Process for the alkylation of paraffins

(30) Priorité: 20.08.1992 FR 9210222; 26.05.1993 FR 9306397
(43) Date de publication de la demande: 23.02.1994
(73) Titulaire: INSTITUT FRANCAIS DU PETROLE, F-92502 Rueil-Malmaison (FR)
(72) Inventeur: Joly, Jean-François, F-75003 Paris (FR); Benazzi, Eric, F-78360 Montesson (FR); Marcilly, Christian, F-78800 Houilles (FR); Pontier, Renaud, F-38200 Vienne (FR); Le Page, Jean-François, F-92500 Rueil Malmaison (FR)

(56) Documents cités:
- FR-A- 857 145
- GB-A- 540 824
- US-A- 2 233 363
- US-A- 2 409 681
- US-A- 2 433 944
- US-A- 3 371 127

## Description

La présente invention concerne un nouveau procédé d'alkylation d'au moins une isoparaffine, de préférence de l'isobutane et/ou l'isopentane, par au moins une oléfine contenant de 2 à 6 atomes de carbone par molécule.

Pour alimenter les moteurs à combustion interne et à allumage commandé, et notamment les moteurs à taux de compression élevé, il est particulièrement intéressant de disposer de carburants à haut indice d'octane, c'est-à-dire comprenant essentiellement des hydrocarbures paraffiniques fortement ramifiés. L'alkylation d'isoparaffines (de préférence l'isobutane et/ou l'isopentane) par les oléfines contenant de 2 à 6 atomes de carbone par molécule permet d'obtenir de tels produits. Cette réaction nécessite la mise en oeuvre de catalyseurs très acides, dans le but notamment de réduire les réactions parasites telles que les réactions de dimérisation et de polymérisation de l'oléfine, qui fournissent des hydrocarbures moins ramifiés et des hydrocarbures insaturés, les réactions de craquage et les réactions de dismutation.

Les procédés industriels existant pour la production d'hydrocarbures par alkylation de l'isobutane par les oléfines utilisent comme catalyseur soit l'acide sulfurique soit l'acide fluorhydrique. Dans ces procédés, le catalyseur acide constitue une phase liquide qui est mise en contact avec le mélange isoparaffine(s)-oléfine(s) liquide pour former une émulsion. Ces procédés sont coûteux et posent d'importants problèmes de rejets polluants ainsi que de sécurité.

US-A- 2409681 décrits un procédé d'alkylation de l'isoparaffine avec des olefines en utilisant un catalyseur specifique. Ce catalyseur est un complex de chlorure d'aluminium/hydrocarbures qui est particulièrement actif dans l'alkylation de l'ethylène ou du propylène.

D'autres procédés existent également qui utilisent des catalyseurs acides hétérogènes tels des tamis moléculaires, des résines macroréticulaires éventuellement associées avec le trifluorure de bore (BF₃), des résines perfluorées, des acides de Lewis et/ou de Brönsted déposés sur différents supports inorganiques, des alumines chlorées, des graphites intercalés par des acides de Lewis et/ou de Brönsted et des anions déposés sur des supports oxydes tels que ZrO₂/SO₄. Parmi ces procédés on peut en distinguer deux types : (i) le catalyseur est mis en suspension dans une phase hydrocarbonée par agitation vigoureuse au sein du réacteur : c'est le cas du réacteur parfaitement agité ; (ii) le catalyseur est en lit fixe dans le réacteur.

Parmi les procédés de type (i), on peut citer les brevets US-A-3879489 et US-A-3855343 ; ce type de mise en oeuvre présente un inconvénient majeur : le catalyseur peut être rapidement détruit par attrition, ce qui pose le problème de l'élimination des fines. Une amélioration de la mise en oeuvre de procédé de type (ii) décrite dans le brevet US-A-3852371, qui propose une injection de la charge isoparaffine(s)-oléfine(s) à différentes hauteurs dans le lit de catalyseur, avec recyclage partiel à ces mêmes hauteurs, ce qui paraît plus complexe à maîtriser. D'autre part, les brevets US-A-3852371 et US-A-5073665, qui font état d'une mise en oeuvre préférée en lit fixe, décrivent le recyclage partiel de l'effluent sortant du réacteur en entrée du réacteur, le brevet US-A-5073665 fait état, dans le cas d'une mise en oeuvre comportant au moins un lit fixe de catalyseur, d'une alimentation en oléfine(s) pour chaque lit fixe de catalyseur.

Le procédé de la présente invention est un procédé d'alkylation dans lequel on traite une charge comprenant d'une part au moins une isoparaffine, de préférence au moins un élément choisi dans le groupe formé par l'isobutane et l'isopentane, de manière encore plus préférée l'isobutane, et d'autre part au moins une oléfine contenant de 2 à 6 atomes de carbone par molécule, en présence d'un catalyseur solide d'alkylation ; ledit procédé comprend :
a) l'introduction et la mise en contact avec le catalyseur présent dans une zone réactionnelle R des composés suivants :
   (i) ladite charge, de préférence introduite au moins à l'entrée de la zone R,
   (ii) une partie de l'effluent liquide décrit en d), de préférence introduite au moins à l'entrée de la zone R,
b) le recyclage d'une partie de l'effluent liquide sortant de la zone réactionnelle R vert l'entrée de ladite zone R,
c) l'introduction d'une autre partie de l'effluent liquide sortant de la zone réactionnelle R dans une zone de séparation isobutane/normal-butane/alkylat S,
d) le recyclage de la majeure partie de l'effluent liquide riche en isobutane de la zone S en zone réactionnelle R,
e) l'obtention d'un alkylat, à titre de produit, de la zone S, et,
f) l'obtention de normal-butane à titre de purge de la zone S.

L'invention est caractérisée en ce que le catalyseur présent dans la zone R est un catalyseur solide choisi parmi les catalyseurs suivants :
* un catalyseur comprenant au moins de l'acide sulfurique imprégné sur un support poreux organique ou minéral, tels les catalyseurs décrits dans les demandes de brevet français FR-A-26 82 891, 26 83 740, 26 83 739 et 26 87 935
* un catalyseur comprenant le mélange contenant d'une part au moins un halogénure de composé choisi dans le groupe formé par l'aluminium et le bore et d'autre part au moins un halogénure d'ammonium quartenaire et/ou halohydrate d'amine tel par exemple que le catalyseur décrit dans la demande de brevet français FR-A-26 86 526,
et en ce que une partie de l'effluent liquide décrit en b) est introduit au moins à l'entrée de la zone R, de préference totalement (composé (iii)).

Le catalyseur préféré de la présente invention comprend au moins de l'acide sulfurique imprégné sur support poreux organique ou minéral.

De préférence, et en particulier dans le cas de la mise en oeuvre préférée du procédé selon l'invention où la zone réactionnelle R fonctionne en lit fluide, le catalyseur est principalement constitué de grains sensiblement sphériques de diamètre compris entre 40 et 400µm, de préférence entre 80 et 150µm.

Selon une mise en oeuvre du procédé selon l'invention, il est possible d'introduire la charge, c'est-à-dire le compose (i) décrit en a), en plusieurs points de la zone R. Ces différents points d'injection du composé (i) sont répartis le long de la zone réactionnelle, et de préférence un de ces points d'injection est l'entrée de ladite zone. Ladite répartition se fait de façon la plus avantageuse pour le déroulement de la réaction, selon les conditions opératoires et les composés présents dans la zone R.

Selon une autre mise en oeuvre du procédé selon l'invention, indépendante ou non de la mise en oeuvre décrite précédemment il est possible d'introduire le composé (ii) décrit en a) en plusieurs points de la zone R. Ces différents points d'injection du composé (ii) sont répartis le long de la zone réactionnelle, mais un de ces points d'injection est toujours l'entrée de ladite zone. Ladite répartition se fait de façon la plus avantageuse pour le déroulement de la réaction, selon les conditions opératoires et les composés présents dans la zone R.

Selon une autre mise en oeuvre préférée du procédé selon l'invention, indépendante ou non des mises en oeuvre décrites précédemment, il est possible d'introduire le composé (iii) décrit en a) en plusieurs points de la zone R. Ces différents points d'injection du composé (iii) sont répartis le long de la zone réactionnelle, et de préférence un de ces points d'injection est l'entrée de ladite zone. Ladite répartition se fait de façon la plus avantageuse pour le déroulement de la réaction, selon les conditions opératoires et les composés présents dans la zone R.

De préférence, les fractions des composés (i) et (ii) décrits en a) qui sont introduites dans la zone R sont mélangées ensemble en partie ou en totalité, de préférence en totalité, avant d'être introduites dans ladite zone.

Dans une autre mise en oeuvre préférée du procédé selon l'invention, la zone réactionnelle fonctionne en lit fluide. Dans ladite mise en oeuvre, le catalyseur est fluidisé par le mélange décrit en a). L'un des avantages de cette mise en oeuvre préférée est que la vitesse du mélange dans la zone réactionnelle R règle la hauteur du lit catalytique. D'autres avantages de ladite mise en oeuvre sont la facilité avec laquelle on peut soutirer ou ajouter du catalyseur dans ladite zone R, l'élimination possible dans le liquide par entraînement des grains de catalyseurs qui ont perdu de la matière par attrition et l'obtention d'une bonne agitation au voisinage des grains de catalyseurs tout en limitant les chocs des grains entre eux.

La température dans la zone réactionnelle R est généralement comprise entre -30 et +5°C, de préférence entre -15 et 0°C, et la pression est telle que tout produit injecté dans la zone R, à quelque niveau d'injection que ce soit, comprenant éventuellement une fraction de composé (i) et/ou éventuellement une fraction de composé (iii) et/ou éventuellement une fraction de composé (ii), est liquide à l'injection dans ladite zone. L'élimination des calories sera réalisée d'une manière préférée par un échangeur de chaleur situé sur le circuit de recycle (étape b), composé (iii)).

De préférence, la charge a été séchée sur tamis moléculaire et hydrogénée sélectivement avant son introduction dans la zone réactionnelle R de manière à éliminer les composés très fortement insaturés susceptibles d'inhiber la phase catalytique.

Généralement, le rapport molaire [somme de (isobutane et/ou isopentane) présent(s) dans les composés (i) et (ii) décrits en a)] / [somme de la ou les oléfine(s) présente(s) dans les composés (i) et (ii) décrits en a)] est compris entre 1 et 100, de préférence entre 3 et 50 et de manière encore plus préférée entre 5 et 15.

Le rapport du débit massique du composé (iii) et de la somme des débits massiques des composés (i) et (ii) est généralement compris entre 2 et 10000, de préférence entre 5 et 1000.

Dans le cas de la mise en oeuvre préférée du procédé selon l'invention où la zone réactionnelle fonctionne en lit fluide, la vitesse de la phase liquide dans ladite zone est généralement comprise entre 0,1 et 50 mm/s selon la densité de grains et le diamètre des grains du catalyseur de façon à assurer la fluidisation. Les réactifs sont introduits de façon à ce que la vitesse spatiale horaire, exprimée en poids d'oléfine(s) introduite(s) par unité de poids du catalyseur et par heure, soit généralement comprise entre 0,001 et 10 h⁻¹, de préférence entre 0,002 et 2 h⁻¹, et de manière encore plus préférée entre 0,015 et 0,1 h⁻¹.

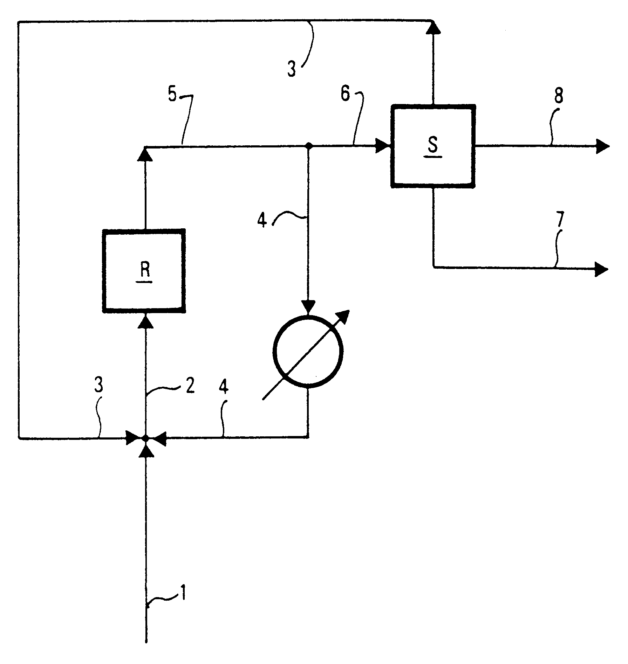

La figure jointe illustre l'invention, et plus précisement une mise en oeuvre du procédé selon l'invention, sans en limiter la portée.

Le mélange en phase liquide, comprenant au moins une isoparaffine, de préférence au moins un élément choisi dans le groupe formé par l'isobutane et l'isopentane, au moins une oléfine contenant de 2 à 6 atomes de carbone par molécule, est introduit par la ligne (1) puis par la ligne (2) après mélange avec les effluents liquides des lignes (3) et (4) dans la zone réactionnelle R.

Un échangeur de chaleur, situé sur la ligne (4), permet d'éliminer la chaleur dégagée par la réaction et de maintenir la température du liquide dans le réacteur à la valeur désirée.

Une partie de l'effluent liquide sortant de la zone R par la ligne (5) est recyclée par la ligne (4) puis par la ligne (2), après mélange avec les effluents liquides des lignes (1) et (3), vers la zone réactionnelle R. La partie de l'effluent liquide sortant de la zone réactionnelle R, et qui n'est pas recyclée vers l'entrée de ladite zone R, est envoyée par la ligne (6) vers une zone de séparation isobutane/normal-butane/alkylat S.

L'alkylat séparé dans la zone S est extrait de l'unité à titre de produit par la ligne (7). Le normal-butane est extrait latéralement de la zone S par la ligne (8) à titre de purge. La fraction liquide riche en isobutane extraite en tête de la zone S est recyclée vers l'entrée de la zone réactionnelle R par la ligne (3).
L'exemple qui suit illustre l'invention sans en limiter la portée.

### Exemple

### (i) préparation du catalyseur

Le catalyseur est préparé en effectuant une imprégnation à sec de 54 g d'une silice caractérisée par un volume poreux total de 1,2 cm³/g, une surface spécifique égale à 55 m²/g, et ayant été calcinée sous air à 150°C pendant 12 heures, par 48 cm³ d'une solution contenant 95% poids d'acide sulfurique de titre 98,7% et 5% poids d'acide trifluorométhanesulfonique de titre 98%. Le catalyseur ainsi obtenu est conservé à l'abri de l'air jusqu'à utilisation.

### (ii) conditions de test catalytique d'alkylation de l'isobutane

Pour alkyler l'isobutane par le butène-l, on utilise un réacteur tubulaire de diamètre interne égal à 4,2 cm et de hauteur égale à 33 cm. Ce réacteur, qui contient 140 g du catalyseur préparé en (i), est alimenté par le bas par une phase liquide dont la vitesse linéaire, dans le réacteur est égale à 1,2 cm/sec, vitesse suffisante pour assurer la fluidisation du catalyseur.

Afin de simuler le recylage de l'isobutane de la zone de séparation S vers l'entrée du réacteur, on utilise comme charge un mélange liquide d'isobutane et de butène-1 contenant 8,5% en poids de butène-1.

Le débit volumique d'injection de la charge décrite ci-dessus est égal à 100 ml/h.

La majeure partie de l'effluent liquide sortant du réacteur est recyclée vers l'entrée dudit réacteur après mélange avec la charge. Pour cela on utilise un débit volumique de recycle égal à 50 l/h. On soutire en continu de l'unité à un débit voisin de 100 ml/h une phase liquide contenant de l'isobutane, du butène-1 non converti et l'alkyle produit. L'alkylat est séparé de ce mélange par vaporisation de l'isobutane.

La température de la phase liquide dans le réacteur est égale à -5°C. Pour maintenir une température de -5°C on utilise un échangeur de chaleur composé d'un serpentin d'un volume de 200 ml situé sur le circuit de recycle et immergé dans un bain refroidi à -12°C.

La composition pondérale de l'alkylat recueille après 200 heures de test continu est la suivante :

| | |
|---|---|
| C₅-C₇ : | 5,5 % |
| C₈ : | 92 % |
| C₉⁺ : | 2,5 % |

La teneur en triméthylpentanes dans la fraction C₈ est égale à 91,5%. La conversion du butène-1 est de environ 99,8%.

## Revendications

1. Procédé d'alkylation dans lequel on traite une charge comprenant d'une part au moins une isoparaffine et d'autre part au moins une oléfine contenant de 2 à 6 atomes de carbone par molécule, en présence d'un catalyseur solide d'alkylation; ledit procédé comprenant:
a) l'introduction et la mise en contact avec le catalyseur présent dans une zone réactionelle R des composés suivants:
(i) ladite charge et
(ii) une partie de l'effluent liquide décrit en d),
b) le recyclage d'une partie de l'effluent liquid sortant de la zone réactionelle R vers l'entrée de ladite zone R,
c) l'introduction d'une autre partie de l'effluent liquide sortant de la zone réactionelle R dans une zone de séparation isobutane/normal-butane/alkylat S,
d) le recyclage de la majeure partie de l'effluent liquide riche en isobutane de la zone S en zone réactionelle R,
e) l'obtention d'un alkylat, à titre de produit, de la zone S, et,
f) l'obtention de normal-butane à titre de purge, de la zone S.
ledit procédé étant caractérisé en ce que le catalyseur est choisi parmi les catalyseurs suivants:
* un catalyseur comprenant au moins de l'acide sulfurique imprégné sur un support poreux organique ou minéral
* un catalyseur comprenant le mélange contenant d'une part au moins un halogénure de composé choisi dans le groupe formé par l'aluminium et le bore et d'autre part au moins un halogénure d'ammonium quaternaire ou un halohydrated'amine, ledit mélange étant imprégné sur un support poreux organique ou minéral et en ce qu'une partie d'effluent liquide décrit en b) est introduite au moins à l'entrée de la zone R (composé (iii)).

2. Procédé selon la revendication 1 dans lequel l'isoparaffine est choisie dans le groupe formé par l'isobutane et l'isopentane.

3. Procédé selon l'une des revendications 1 ou 2 dans lequel le composé (i) est introduit dans la zone R en plusieurs points, dont l'entrée de ladite zone R.

4. Procédé selon l'une des revendications 1 à 3 dans lequel le composé (iii) est introduit dans la zone R en plusieurs points, dont l'entrée de ladite zone R.

5. Procédé selon l'une des revendications 1 à 4 dans lequel les fractions des composés (i) et (ii) décrits en a) qui sont introduites à l'entrée de la zone R sont mélangées ensemble, en totalité ou en partie, avant d'être introduites dans ladite zone.

6. Procédé selon l'une des revendications 1 à 5 dans lequel le composé (iii) est introduit totalement à l'entrée de la zone R.

7. Procédé selon l'une des revendications 1 à 6 dans lequel le catalyseur est principalement constitué de grains sensiblement sphériques de diamètre compris entre 80 et 400µm.

8. Procédé selon l'une des revendications 1 à 7 dans lequel la zone réactionnelle fonctionne en lit fluide.

9. Procédé selon l'une des revendications 1 à 8 dans lequel le rapport du débit massique du composé (iii) et de la somme des débits massiques des composés (i) et (ii) est généralement compris entre 2 et 10 000.

10. Procédé selon l'une des revendications 1 à 9 dans lequel le catalyseur utilisé comprend au moins de l'acide sulfurique imprégné sur un support poreux organique ou minéral.

## Claims

1. Alkylation process, in which treatment takes place of a charge incorporating on the one hand at least one isoparaffin and on the other at least one olefin containing 2 to 6 carbon atoms per molecule, in the presence of a solid alkylation catalyst, said process comprising:
a) the introduction and contacting with the catalyst present in a reaction zone R of the following compounds:
(i) the said charge,
(ii) part of the liquid effluent described in d),
b) the recycling of pant of the liquid effluent passing out of the reaction zone R to the intake of said zone R,
c) the introduction of another part of the liquid effluent passing out of the reaction zone R into an isobutane/n-butane/alkylate separation zone S,
d) the recycling of the major part of the isobutane-rich liquid effluent from the zone S to the reaction zone R,
e) the obtaining of an alkylate, as product, from the zone S and
f) the obtaining of n-butane, as purge, from the zone S,
said process being characterized in that the catalyst is chosen from among the following catalysts:
a catalyst incorporating at least sulphuric acid impregnated on an organic or mineral porous support,
a catalyst incorporating the mixture containing on the one hand at least one halide of a compound chosen from within the group formed by aluminium and boron and on the other at least one quaternary ammonium halide or an amine halohydrate, said mixture being impregnated on an organic or mineral porous support, and in that a part of the liquid effluent described in b) in introduced at least at the inlet of the zone R. (compound (iii)).

2. Process according to claim 1, wherein the isoparaffin is chosen from within the group formed by isobutane and isopentane.

3. Process according to either of the claims 1 or 2, wherein the compound (i) is introduced into the zone R at several points, including the intake of the zone R.

4. Process according to any one of the claims 1 to 3, wherein the compound (iii) is introduced into the zone R at several points, including the intake of said zone R.

5. Process according to any one of the claims 1 to 4, wherein the fractions of the compounds (i) and (ii) described in a) and which are introduced at the intake of the zone R are mixed together, totally or partly, before being introduced into the said zone.

6. Process according to any one of the claims 1 to 5, wherein the compound (iii) is totally introduced at the intake of the zone R.

7. Process according to any one of the claims 1 to 6, wherein the catalyst is mainly constituted by substantially spherical grains with a diameter between 80 and 400 µm.

8. Process according to any one of the claims 1 to 7, wherein the reaction zone functions as a fluid bed.

9. Process according to any one of the claims 1 to 8, wherein the ratio of the mass flow rate of the compound (iii) and the sum of the mass flow rates of the compounds (i) and (ii) is generally between 2 and 10,000.

10. Process according to any one of the claims 1 to 9, wherein the catalyst used comprises at least sulphuric acid impregnated on an organic or mineral porous support.

## Patentansprüche

1. Verfahren zur Alkylierung, worin man eine Charge, welche einesteils wenigstens ein Isoparaffin und andernteils wenigstens ein 2 bis 6 Kohlenstoffatome pro Molekül enthaltendes Olefin in Gegenwart eines festen Alkylierungskatalysators behandelt, wobei das Verfahren umfaßt:
a) das Einführen und das mit dem in einer Reaktionszone R vorhandenen Katalysator in Kontakt bringen von folgenden Verbindungen:
(i) die Charge und
(ii) einen Teil des in d) beschriebenen flüssigen Abstromes,
b) die Rückführung eines Teiles des flüssigen Abstromes, der die Reaktionszone R verläßt, zum Eingang der Zone R,
c) die Einführung eines weiteren Teiles des flüssigen Abstromes, welcher die Reaktionzone R verläßt, in eine Auftrennungszone S für i-Butan/n-Butan/Alkylat,
d) die Rückführung des größten Teiles des an Isobutan reichen flüssigen Abstromes der Zone S zur Reaktionszone R,
e) das Erhalten eines Alkylats als Produkt aus der Zone S und,
f) das Erhalten von n-Butan als Spülung aus der Zone S,
wobei das Verfahren dadurch gekennzeichnet ist, daß der Katalysator unter den folgenden Katalysatoren ausgewählt wird:
* einem Katalysator, der wenigstens auf einem organischen oder mineralischen porösen Träger imprägnierte Schwefelsäure umfaßt,
* einem Katalysator, welcher die Mischung umfaßt, welche einerseits wenigstens ein Halogenid einer Verbindung enthält, die aus der durch Aluminium und Bor gebildeten Gruppe ausgewählt ist und anderenteils wenigstens ein quaternäres Ammoniumhalogenid oder ein Aminhalohydrat enthält, wobei die Mischung auf einem organischen oder mineralischen porösen Träger imprägniert ist
und dadurch, daß ein Teil des in b) beschriebenen flüssigen Abstromes wenigstens in den Eingang der Zone R eingeführt wird (Verbindung (iii)).

2. Verfahren nach Anspruch 1, worin das Isoparaffin aus der durch Isobutan und Isopentan gebildeten Gruppe ausgewählt ist.

3. Verfahren nach einem der Ansprüche 1 oder 2, worin die Verbindung (i) in die Zone R an mehreren Stellen mit dem Eingang der Zone R eingeführt wird.

4. Verfahren nach einem der Ansprüche 1 bis 3, worin die Verbindung (iii) in die Zone R an mehreren Stellen mit dem Eingang der Zone R eingeführt wird.

5. Verfahren nach einem der Ansprüche 1 bis 4, worin die Fraktionen der in a) beschriebenen Verbindungen (i) und (ii), welche am Eingang der Zone R eingeführt werden, ganz oder teilweise miteinander vermischt werden, bevor sie in die Zone eingeführt werden.

6. Verfahren nach einem der Ansprüche 1 bis 5, worin die Verbindung (iii) vollständig am Eingang der Zone R eingeführt wird.

7. Verfahren nach einem der Ansprüche 1 bis 6, worin der Katalysator hauptsächlich aus im wesentlichen sphärischen Kügelchen mit einem Druchmesser, der zwischen einschließlich 80 und 400 µm enthalten ist, besteht.

8. Verfahren nach einem der Ansprüche 1 bis 7, worin die Reaktionszone mit einem Fließbett arbeitet.

9. Verfahren nach einem der Ansprüche 1 bis 8, worin das Verhältnis von Mengendurchfluß der Verbindung (iii) und der Summe der Mengendurchflüsse der Verbindungen (i) und (ii) im allgemeinen zwischen einschließlich 2 und 10.000 enthalten ist.

10. Verfahren nach einem der Ansprüche 1 bis 9, worin der verwendete Katalysator wenigstens auf einem organischen oder mineralischen porösen Träger imprägnierte Schwefelsäure umfaßt.
